# EUROPÄISCHE PATENTANMELDUNG

(11) **EP 4 813 415 A1**
(43) Veröffentlichungstag der Anmeldung: **30.09.2026**
(21) Anmeldenummer: 25166790.3
(22) Anmeldetag: 27.03.2025
(51) Int. Cl.: A61M 5/158, A61M 5/14, A61M 39/10, A61M 39/00

(54) **INFUSIONSSET FÜR EINE INFUSIONSVORRICHTUNG**

(71) Anmelder: mylife Diabetes Care AG, 3400 Burgdorf (CH)
(72) Erfinder: Ziemendorff, Etienne, 8050 Zürich (CH); Stucki, Janick, 3400 Burgdorf (CH); Da Eira, Natalie, 1299 Crans (CH)
(74) Vertreter: Finklenburg, Susanne

(57) **Zusammenfassung**

Die Erfindung betrifft ein Infusionsset, wobei das Infusionsset der Zuführung eines oder mehreren flüssigen Produkte dient. Das Infusionsset umfasst einen Basisteil (1) mit einer Kanüle (1a) zur Zuführung des flüssigen Produkts in den Körper des Patienten. Das Infusionsset umfasst des Weiteren einen Verbindungsteil (2) und einen ersten (3) und zweiten Konnektorteil (4), wobei der erste Konnektorteil (3) und der zweite Konnektorteil (4) an den Verbindungsteil (2) derart ankoppelbar und abkoppelbar sind, dass jeweils eine lösbare Verbindung in eine Richtung quer zur Längsachse der Kanüle (1a) erfolgt.

## Beschreibung

Die Erfindung betrifft ein Infusionsset für eine Infusionsvorrichtung, wobei das Infusionsset der Zuführung eines oder mehreren flüssigen Produkte, insbesondere flüssigen Medikamente von einer Infusionsvorrichtung in den Körper dient.

Der Begriff "Medikament" umfasst hier jede fliessfähige medizinische Formulierung, welche geeignet ist zur kontrollierten Verabreichung durch ein Mittel wie eine Kanüle oder Nadel, insbesondere eine Hohlnadel hindurch, beispielsweise umfassend eine Flüssigkeit, eine Lösung, ein Gel, oder eine feine Suspension, welche einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzymen (z.B. Hydrolasen, Lyasen) und weitere Substanzen sowohl in fester (suspendierter) oder flüssiger Form aber auch Polysaccaride (z.B. Glycosaminoglycan), Vaccine, DNS oder RNS oder Oglionukleotide, Antikörper oder Teile von Antikörpern sowie geeignete Basis-, Hilfs- und Trägerstoffe.

Bei Patienten, welche einen regelmässigen Bedarf an einem flüssigen Medikament haben, kann dem Körper die erforderliche Medikamentenmenge in flüssiger Form mittels einer über einen längeren Zeitraum im subkutanen Gewebe angeordneten Kanüle zugeführt werden. Hierzu wird auf der Haut des Patienten ein Infusionsset mit einer Kanüle aus einem weichen Material oder aus einem steifen Material derart befestigt, dass die Kanüle durch die Haut bis in das subkutane Gewebe eindringt. Die Verabreichung der erforderlichen Medikamentenmenge in flüssiger Form erfolgt somit von einer Infusionsvorrichtung, insbesondere von einer Infusionspumpe, insbesondere bevorzugt von einer automatischen Infusionspumpe über eine Zuführungsleitung zum Patienten, wobei die in dem Infusionsset angeordnete Kanüle den Patienten mit dem flüssigen Medikament versorgt.

Aus dem Stand der Technik sind Infusionssets bekannt. Das Dokument EP2026865B1 offenbart ein Infusionsset, welches ein erstes Bauteil mit einer Kanüle aufweist, wobei die Kanüle zum Eindringen durch die Haut bis in das subkutane Gewebe dient, um ein flüssiges Medikament an einen Patienten zu verabreichen. Zudem weist das Infusionsset ein zweites Bauteil auf, welches relativ zu dem ersten Bauteil um eine parallel zur Längsachse der Kanüle verlaufende Rotationsachse drehbar ist. Ferner weist das Infusionsset einen Konnektorteil auf, an welchem eine Zuführungsleitung, insbesondere ein Schlauch angebracht ist. Die Zuführungsleitung kann mit einer Infusionsvorrichtung, insbesondere mit einer Infusionspumpe verbunden werden. Somit kann durch das Infusionsset das flüssige Medikament von der Infusionsvorrichtung dem Patienten verabreicht werden. Der Konnektorteil kann quer zur Längsachse der Kanüle an den zweiten Teil gekoppelt oder von dem zweiten Teil abgekoppelt werden. Durch diese Anordnung kann der Tragekomfort verbessert werden.

Es ist eine Aufgabe der Erfindung ein alternatives Infusionsset bereitzustellen.

Diese Aufgabe wird durch den Gegenstand des unabhängigen Anspruchs 1 gelöst. Vorteilhafte Ausführungsformen sind in den abhängigen Ansprüchen definiert.

In die distale Richtung bedeutet bei dem erfindungsgemässen Infusionsset die Seite, an welcher ein Heftpflaster angebracht sein kann, um das Infusionsset auf der Haut eines Patienten anzubringen. In die proximale Richtung bedeutet bei dem erfindungsmässen Infusionsset die der distalen Seite entgegengesetzten Seite.

Das erfindungsgemässe Infusionsset kann an eine Infusionsvorrichtung, insbesondere an eine Infusionspumpe, besonders bevorzugt an eine automatische Infusionspumpe anbringbar sein. Infusionsvorrichtungen können der kontinuierlichen Verabreichung von Infusionen, insbesondere von Medikamenten in flüssiger Form dienen. Bei der Infusionsvorrichtung kann es sich um eine wiederverwendbare Infusionsvorrichtung handeln, wobei ein neuer mit einem flüssigen Medikament vorgefüllter Container in die wiederverwendbare Infusionsvorrichtung einsetzbar ist. Andererseits kann es sich bei der Infusionsvorrichtung um eine einmalig verwendbare Infusionsvorrichtung handeln, wobei die Infusionsvorrichtung weggeworfen wird, wenn ein mit dem flüssigen Medikament vorgefüllter Container leer ist.

Das Infusionsset umfasst einen Basisteil zum Anbringen auf der Haut eines Patienten, wobei der Basisteil eine proximale Seite und eine distale Seite aufweist. Ferner weist der Basisteil eine Kanüle zur Zuführung des flüssigen Medikaments in den Körper des Patienten auf. Die Kanüle ist auf der distalen Seite des Basisteils vorgesehen. Ferner umfasst das Infusionsset ein Verbindungsteil. Der Verbindungsteil ist an der proximalen Seite des Basisteils untrennbar vorgesehen. Der Verbindungsteil und das Basisteil sind relativ zueinander um eine parallel zur Längsachse der Kanüle verlaufenden Rotationsachse (R) drehbar angeordnet. Durch diese Anordnung kann der Tragekomfort verbessert werden. Ferner kann das Infusionsset einen ersten Konnektorteil mit einer ersten Zuführungsleitung, insbesondere einem ersten Schlauch umfassen. Die erste Zuführungsleitung kann zur Verbindung mit der ersten Infusionsvorrichtung dienen. Ferner weist der erste Konnektorteil eine erste Zuführungskanüle zur Verbindung mit dem Verbindungsteil auf.

Ferner kann das Infusionsset einen Kappenteil zum Halten des Infusionssets durch den Patienten oder durch eine Insertionsvorrichtung oder Einstechhilfe umfassen. Das Infusionsset kann in einer ersten Ausführungsform der Erfindung mit einer weichen Kanüle, wobei die Kanüle aus einem weichen oder flexiblen Material, insbesondere aus einem Polymer ausgebildet sein kann, oder in einer zweiten Ausführungsform der Erfindung mit einer steifen Kanüle, wobei die Kanüle aus Stahl oder aus einem alternativen steifen Material ausgebildet sein kann, ausgestattet sein.

In einer ersten Ausführungsform des Infusionssets kann der Kappenteil des Infusionssets eine steife Nadel, wobei die Nadel aus Stahl oder aus einem alternativen steifen Material ausgebildet sein kann, aufweisen. Der Kappenteil kann mit dem Verbindungsteil oder mit dem Basisteil lösbar verbunden werden oder sein. Die Verbindung kann vorzugsweise als Rastverbindung ausgebildet sein. Alternative Verbindungen können vorgesehen sein. Der Kappenteil kann mit dem Verbindungsteil oder mit dem Basisteil derart verbunden, insbesondere verrastet sein, dass die steife Nadel konzentrisch in einer weichen Kanüle des Basisteils angeordnet sein kann. Die weiche Kanüle kann der Zuführung eines flüssigen Medikaments in den Körper eines Patienten dienen. Der Kappenteil kann an einer proximalen Seite des Verbindungsteils oder des Basisteils angeordnet werden oder sein. Die konzentrische Anordnung der weichen Kanüle und der steifen Nadel kann der Führung der weichen Kanüle dienen, wenn die weiche Kanüle des Basisteils in die Haut des Patienten eingestochen wird. Dabei kann die distale Spitze der steifen Nadel über das distale Ende der weichen Kanüle ragen. Ferner kann an der distalen Seite des Basisteils ein Heftpflaster angeordnet sein. Das Heftpflaster kann dem Befestigen des Infusionssets auf der Haut des Patienten dienen. Um das Infusionsset auf der Haut des Patienten anzubringen, kann somit der mit dem Kappenteil direkt oder indirekt verbundene Basisteil mittels Heftpflaster auf die Haut des Patienten geklebt werden, wobei vorgängig eine Schutzfolie von einer Klebeseite des Heftpflasters entfernt werden kann. Dabei kann die mit der festen Nadel aufgenommene weiche Kanüle durch die Haut des Patienten und in das subkutane Gewebe des Patienten eindringen. Um das Infusionsset mit einer Infusionsvorrichtung zu verbinden, kann zuerst der Kappenteil mit der Nadel von dem Basisteil abgenommen werden und ein erster Konnektorteil, welcher die erste Zuführungskanüle umfasst, an den Verbindungsteil des Infusionssets angebraucht werden. Die Verbindung zwischen dem Verbindungsteil und dem ersten Konnektorteil kann als Rastverbindung und/oder als Eingriffsverbindung ausgebildet sein. Alternative Verbindungen können vorgesehen sein. Die Verbindung zwischen dem Verbindungsteil und dem ersten Konnektorteil ist lösbar ausgebildet. Der Verbindungsteil und der erste Konnektorteil können ein Rastelement und ein erstes korrespondierendes Rastgegenelement aufweisen. Der Verbindungsteil und der erste Konnektorteil können Führungen aufweisen, so dass der Verbindungsteil und der erste Konnektorteil in Eingriff gelangen. Die erste Zuführungskanüle und der erste Konnektorteil können zweistückig ausgebildet sein. Der erste Zuführungskanüle kann mit dem ersten Konnektorteil verschweisst und/oder verklebt sein. Alternativ kann die erste Zuführungskanüle und der erste Konnektorteil einstückig, insbesondere aus dem gleichen Material ausgebildet sein. Die erste Zuführungsleitung des ersten Konnektorteils kann einen Adapter aufweisen. Die erste Zuführungsleitung kann mit dem Adapter verschweisst und/oder verklebt sein. Der Adapter des ersten Konnektorteils kann lösbar an ein entsprechendes korrespondierendes Verbindungselement der ersten Infusionsvorrichtung angebracht werden. Somit kann eine Fluidverbindung von der Infusionsvorrichtung über das Infusionsset zu dem Patienten entstehen.

In einer zweiten Ausführungsform des Infusionssets kann das Infusionsset einen alternativ ausgebildeten Kappenteil umfassen. Der Kappenteil weist keine steife Nadel auf. Der Basisteil kann eine steife Kanüle umfassen. Die steife Kanüle kann der Zuführung eines flüssigen Medikaments in den Körper eines Patienten dienen. Der Kappenteil kann mit dem Verbindungsteil oder mit dem Basisteil lösbar verbunden werden oder sein. Die Verbindung kann vorzugsweise als Rastverbindung ausgebildet sein. Alternative Verbindungen können vorgesehen sein. Der Kappenteil kann an einer proximalen Seite des Verbindungsteils oder des Basisteils angeordnet werden oder sein. An der distalen Seite des Basisteils kann ein Heftpflaster zur Befestigung des Infusionssets auf der Haut des Patienten vorgesehen sein. Um das Infusionsset auf der Haut des Patienten anzubringen, kann der mit dem Kappenteil direkt oder indirekt verbundene Basisteil mittels Heftpflaster auf die Haut des Patienten geklebt werden, wobei vorgängig eine Schutzfolie von einer Klebeseite des Heftpflasters entfernt werden kann. Dabei kann die steife Kanüle des Basisteils in das subkutane Gewebe des Patienten eindringen. Danach kann der Kappenteil von dem Verbindungsteil oder von dem Basisteil entfernt werden, um den ersten Konnektorteil mit dem Verbindungsteil des Infusionssets und der ersten Infusionsvorrichtung, wie bereits oben beschrieben, lösbar zu verbinden.

Die erste und die zweite Ausführungsform des Infusionssets können vorzugsweise ein lösbares Schutzelement aufweisen. Das Schutzelement kann dem Schutz vor Stichverletzungen durch die steife Kanüle oder durch die steife Nadel des entsprechenden Infusionssets dienen. Das Schutzelement kann zudem vor Stickverletzungen durch die erste Verbindungskanüle des Infusionssets dienen. Das Schutzelement kann vorzugsweise hülsenförmig ausgebildet sein. Alternative Ausgestaltungen des Schutzelements können vorgesehen sein. An einer Mantelaussenfläche des Schutzelements können vorzugsweise eine oder mehrere Längsrippen zum besseren Greifen des Schutzelements vorgesehen sein. Das Schutzelement kann mit einem Kraftschluss und/oder mit einem Formschluss mit dem Basisteil und/oder dem Kappenteil und/oder dem ersten Konnektorteil lösbar verbunden sein, um den Patienten vor einer Stichverletzung zu schützen.

Das erfindungsgemässe Infusionsset kann einen zweiten Konnektorteil umfassen. Der zweite Konnektorteil kann in einer Ausführungsform der Erfindung als ein ergänzender Teil zu dem ersten Konnektorteil ausgebildet sein, so dass der erste und der zweite Konnektorteil im Wesentlichen den Basisteil verdecken, wenn der erste und der zweite Konnektorteil in Eingriff sind. Der zweite Konnektorteil kann in einer zweiten Ausführungsform der Erfindung ebenfalls als ein ergänzender Teil zu dem ersten Konnektorteil ausgebildet sein und zusätzlich analog dem ersten Konnektorteil mit einer zweiten Zuführungsleitung, insbesondere einem zweiten Schlauch zur Verbindung mit einer zweiten Infusionsvorrichtung und mit einer zweiten Zuführungskanüle zur Verbindung mit dem Verbindungsteil ausgestattet sein. Der erste und der zweite Konnektorteil können analog der ersten Ausführungsform der Erfindung ebenfalls im Wesentlichen den Basisteil verdecken, wenn der erste und der zweite Konnektorteil in Eingriff sind und wobei zwei flüssige Produkte, insbesondere zwei flüssige Medikamente von den beiden Infusionsvorrichtungen in den Körper zugeführt werden kann.

Wenn zwei flüssige Produkte, insbesondere zwei Medikamente gleichzeitig, hintereinander und/oder bei entsprechendem Bedarf in den Körper verabreicht werden, kann eine Krankheit gezielter therapiert werden.

Beispielsweise kann zur Behandlung von Diabetes Insulin und Glucagon oder Analogon (z.B. Dasiglucagon) durch zwei Infusionsvorrichtung durch das erfindungsgemässe Infusionsset verabreicht werden. Glucagon wirkt antagonistisch zu Insulin. Insulin senkt den Blutzucker, wobei bei einer drohenden oder bei einer bestehenden Hypoglykämie sofort bzw. notfallmässig Glucagon durch die zweite Infusionsvorrichtung über den zweiten Konnektorteil dem Patienten verabreicht werden sollte. Diese Hypoglykämie tritt in erster Linie als Nebenwirkung einer Diabetesbehandlung durch Insulin auf. Denn wenn die zugeführte Menge an Insulin grösser ist als der Bedarf, sinkt der Blutzuckerspiegel zu stark ab, was mit einer sofortigen Zufuhr von Glucagon behandelt werden muss. Durch Messung des Blutzuckers des Patienten, beispielsweise durch einen Sensor können die beiden Infusionsvorrichtung derart gesteuert werden, dass bei Bedarf Insulin oder Glucagon durch das Infusionsset verabreicht wird.

Beispielsweise kann eine verbesserte Behandlung von Diabetes auch durch eine gleichzeitige Verabreichung von Insulin und beispielsweise Amylin-Analogon, GLP-1-Agonist oder SGLT2-Inhibitor durch das erfindungsgemässe Infusionsset erreicht werden. Dabei kann neben der Senkung des Blutzuckers eine gleichzeitige Verlangsamung der Magenentleerung, eine Förderung des Sättigungsgefühls, eine Förderung der Insulinausschüttung, eine Verringerung des Hungergefühls, eine Förderung der Glukoseausschüttung, eine Verbesserung der Herz- und Nierenfunktion und/oder eine Verlangsamung der Magenentleerung erzielt werden.

Es können auch andere Krankheiten, beispielsweise Adipositas, Parkinson, Immun- oder Autoimmunerkrankungen, oder Schmerzen durch eine gleichzeitige oder nachfolgende Verabreichung von zwei flüssigen Produkten, insbesondere zwei Medikamenten gezielter therapiert werden. Dabei können beispielsweise GL1-1-Agonist, Amylin-Analogon, Levodopa bzw. Carbidopa, Apomophin, Opioide, Lokalanästhetikum, Biologika-Kombinationen und/oder Kortikosteroide gleichzeitig oder nachfolgenden dem Patienten durch das erfindungsgemässe Infusionsset abgegeben werden.

Der erste Konnektorteil und der zweite Konnektorteil des erfindungsgemässen Infusionssets sind derart an den Verbindungsteil ankoppelbar und abkoppelbar, dass jeweils eine lösbare Verbindung in eine Richtung quer zur Längsachse der Kanüle erfolgt. Durch diese Anordnung können die Schmerzen, welche durch die eingestochene Kanüle des Basisteils verursacht werden, verringert werden, da während der An- und Abkoppelung kein oder ein verringerter Druck auf die Einstichstelle wirkt.

Ferner umfasst der Verbindungsteil des Infusionssets ein Septum, welches einen parallel zur Längsachse der Kanüle verlaufenden Kanal aufweist, um eine Fluidverbindung zwischen dem Verbindungsteil und der Kanüle in dem Basisteil zu bilden. Zudem erstreckt sich das Septum quer zur Längsachse der Kanüle, so dass die erste Zuführungskanüle des ersten Konnektorteils das Septum durchstossen kann, um eine Fluidverbindung zwischen dem ersten Konnektorteils und dem Kanal des Verbindungsteils zu bilden. Das Septum kann vorzugsweise T-förmig, insbesondere T-förmig in dem Verbindungsteil ausgebildet sein. Das Septum kann ein proximales Ende des Kanals verschliesst. Das Septum ist derart ausgebildet, dass die Zuführungskanüle des ersten Konnktorteils das Septum durchstechen kann. In jeder relativen Drehposition des Verbindungsteils zu dem Basisteil um die Rotationsachse (R) kann die Fluidverbindung zwischen dem ersten Konnektorteil und dem Kanal des Verbindungsteils gebildet sein.

An den Verbindungsteil des Infusionssets kann ein erstes zweites Konnektorteil, welches nur zur zumindest teilweisen Verdeckung des Basisteils dient, an- und abgekoppelt werden. Bei Bedarf kann das erste zweite Konnektorteil durch ein zweites Konnektorteil, welches zur zusätzlichen Verabreichung eines zweiten flüssigen Produkts, insbesondere eines zweiten flüssigen Medikaments in den Körper dient, ersetzt werden.

Das zweiten Konnektorteil kann eine zweite Zuführungskanüle zur Verbindung mit dem Verbindungsteil und eine zweite Zuführungsleitung zur Verbindung mit einer zweiten Infusionsvorrichtung aufweisen, wobei das Septum des Verbindungsteils sich quer zur Längsachse der Kanüle erstreckt, so dass die zweite Zuführungskanüle des zweiten Konnektorteils das Septum durchstossen kann, um eine Fluidverbindung zwischen dem zweiten Konnektorteils und dem Kanal des Verbindungsteils zu bilden.

Der Basisteil und das Verbindungsteil können bevorzugt relativ zueinander um die parallel zur Längsachse der Kanüle verlaufenden Rotationsachse (R) um 360° oder um weniger als 360° drehbar angeordnet sind. Die relative Drehung kann in beide Rotationsrichtungen um die Rotationsachse (R) erfolgen. Der erste und der zweite Konnektorteil können in einer beliebigen relativen Drehposition zwischen dem Basisteil und dem Verbindungsteil an den Verbindungsteil angebracht werden. Bei einer möglichen 360° relativen Drehung um die Rotationsachse (R) ist eine maximale Bewegungsfreiheit des Patienten gewährleistet. Bei einer weniger als 360° relativen Drehung um die Rotationsachse (R) wird ein unerwünschtes Aufwickeln der Zuführungsleitung oder der Zuführungsleitungen verhindert.

Der erste Konnektorteil und der zweite Konnektorteil können an den Verbindungsteil derart ankoppelbar und abkoppelbar sein, dass eine lösbare axialfeste und/oder eine lösbare drehfeste Verbindung zwischen dem ersten Konnektorteil und dem Verbindungsteil und zwischen dem zweiten Konnektorteil und dem Verbindungsteil erfolgen kann.

Das Verbindungsteil kann vorzugsweise ein Rastelement aufweisen, welches mit einem an dem ersten Konnektorteil vorgesehenen ersten korrespondierenden Rastgegenelement eine lösbare, insbesondere eine lösbare axialfeste Verbindung bilden kann und welches mit einem an dem zweiten Konnektorteil vorgesehenen zweiten korrespondierenden Rastgegenelement eine lösbare, insbesondere eine lösbare axialfeste Verbindung bilden kann. Diese lösbare, insbesondere diese lösbare axialfeste Verbindung kann als Rastverbindung ausgebildet sein. Alternativ oder zusätzlich kann die lösbare, insbesondere die lösbare axialfeste Verbindung als Schnappverbindung, Bajonettverbindung, Hakenverbindung oder Schraubverbindung ausgebildet sein. Somit kann insbesondere gewährleistet werden, dass die Verbindung zwischen dem ersten Konnektorteil bzw. dem zweiten Konnektorteil und dem Verbindungsteil zu jedem beliebigen Zeitpunkt eingegangen und gelöst werden kann. Die Rastverbindung dient insbesondere dazu, dass der erste und der zweite Konnektorteil lösbar an den Verbindungsteil ankoppelbar und abkoppelbar sind. Die lösbare Verbindung kann als form- und/oder kraftschlüssige, insbesondere als formschlüssige Verbindung ausgebildet sein. Die lösbare Verbindung kann bevorzugt als lösbare axialfeste Verbindung ausgebildet sein.

Das Rastelements des Verbindungsteils kann in einer bevorzugten Ausführungsform der Erfindung als Ringnut ausgebildet sein und das erste Rastgegenelement des ersten Konnektorteils und das zweite Rastgegenelement des zweiten Konnektorteils können als Rastringe ausgebildet sein, welche in die Ringnut einrasten können. Die Rastverbindungen können als lösbare axialfeste Verbindung ausgebildet sein. Die Rastverbindung dient insbesondere dazu, dass einerseits der erste und der zweite Konnektorteil axialfest an den Verbindungsteil angekoppelt werden können und andererseits von dem Verbindungsteil entfernt oder abgekoppelt werden können. Somit können das erste und das zweite Konnektorteil über eine Rastverbindung an den Verbindungsteil ankoppelbar und abkoppelbar sein, wobei wenn das erste und/oder das zweite Konnektorteil an den Verbindungsteil angekoppelt ist, das erste und/oder das zweite Konnektorteil axialfest mit dem Verbindungsteil verbunden sind.

Das Verbindungsteil kann insbesondere ein Führungselement aufweisen, welches mit einem an dem ersten Konnektorteil vorgesehenen ersten korrespondierenden Führungsgegenelement in Eingriff gelangen kann und welches mit einem an dem zweiten Konnektorteil vorgesehenen zweiten korrespondierenden Führungsgegenelement in Eingriff gelangen kann. Die Führungseingriffe können als lösbare Verbindung ausgebildet sein. Diese Führungseingriffe dienen insbesondere dazu, dass der erste und der zweite Konnektorteil lösbar an den Verbindungsteil ankoppelbar und abkoppelbar sind. Somit kann insbesondere gewährleistet werden, dass die Verbindung zwischen dem ersten Konnektorteil bzw. dem zweiten Konnektorteil und dem Verbindungsteil zu jedem beliebigen Zeitpunkt eingegangen und gelöst werden kann. Die lösbare Verbindung kann als lösbare drehfeste Verbindung ausgebildet sein. Der erste und der zweite Konnektorteil können bevorzugt derart in Eingriff mit dem Verbindungsteil sein, dass sie formschlüssig und/oder kraftschlüssig, insbesondere formschlüssig lösbar ineinandergreifen können. Die Ankoppelung und Abkoppelung zwischen dem ersten bzw. dem zweiten Konnektorteil und dem Verbindungsteil kann derart bevorzugt erfolgen, dass jeweils eine lösbare drehfeste Verbindung um die Rotationsachse (R) erfolgt.

Das Führungselement des Verbindungsteils kann in einer bevorzugten Ausführungsform der Erfindung zumindest teilweise polygonförmig ausgebildet sind. Ferner können das erste Führungsgegenelement des ersten Konnektorteils und das zweite Führungsgegenelement des zweiten Konnektorteils als korrespondierender Teil zu diesem teilweisen polygonförmigen Verbindungsteil ausgebildet sein. Das Verbindungsteil kann einen polygonförmigen Querschnitt aufweisen. Das Polygon kann n-Ecken aufweisen. Das Polygon kann vorzugweise als regelmässiges Polygon ausgebildet sein. Alternativ kann das Polygon unregelmässig ausgebildet sein. Die Seiten des Polygons können als Führung dienen. Die Seiten des Polygons können parallel und/oder schräg zur Längsachse der Kanüle ausgebildet sein.

Der Basisteil kann in einer bevorzugten Ausführungsform der Erfindung kreisförmig ausgebildet sein. Wenn der erste und der zweite Konnektorteil beispielsweise formschlüssig lösbar ineinandergreifen, können die ineinandergreifenden Konnektorteile den Basisteil im Wesentlichen verdecken. Die ineinandergreifenden Konnektorteile können zusammen einen Kreis oder eine Halbkugel bilden. Der erste und der zweite Konnektorteil können kappenförmig ausgebildet sein.

Die Erfindung wird im Folgenden anhand von mehreren Figuren beschrieben. Die hierbei offenbarten Merkmale bilden je einzeln und in jeglicher Merkmalskombination die Erfindung vorteilhaft weiter. Es zeigen:
- Figur 1:: eine erste perspektivische Draufsicht auf eine erfinderische Anordnung, wobei ein erster (3) und ein zweiter Konnektorteil (4) von einem Verbindungsteil (2) entfernt sind.
- Figur 2:: eine zweite perspektivische Draufsicht auf die Anordnung gemäss Figur 1, wobei der erste (3) und der zweite Konnektorteil (4) von dem Verbindungsteil (2) entfernt sind.
- Figur 3:: eine perspektivische Draufsicht auf die Anordnung gemäss den Figuren 1 und 2, wobei der erste Konnektorteil (3) mit dem Verbindungsteil (2) verbunden ist und der zweite Konnektorteil (4) von dem Verbindungsteil (2) entfernt ist.
- Figur 4:: eine perspektivische Draufsicht auf die Anordnung gemäss den Figuren 1 und 2, wobei der erste (3) und der zweite Konnektorteil (4) mit dem Verbindungsteil (2) verbunden sind.
- Figur 5:: eine Längsschnittansicht der Anordnung gemäss den Figuren 1 und 2, wobei der erste (3) und der zweite Konnektorteil (4) gemäss der Figur 4 mit dem Verbindungsteil (2) verbunden sind.

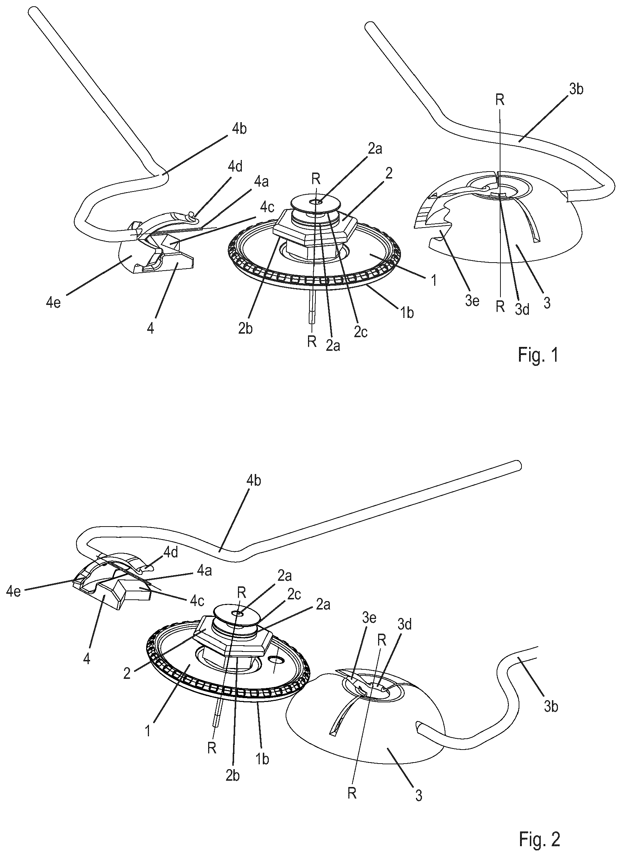

In der Figur 1 ist eine erste perspektivische Draufsicht und in der Figur 2 ist eine zweite perspektivische Draufsicht auf ein erfindungsgemässe Infusionsset dargestellt, wobei ein erster (3) und ein zweiter Konnektorteil (4) von einem Verbindungsteil (2) entfernt sind.

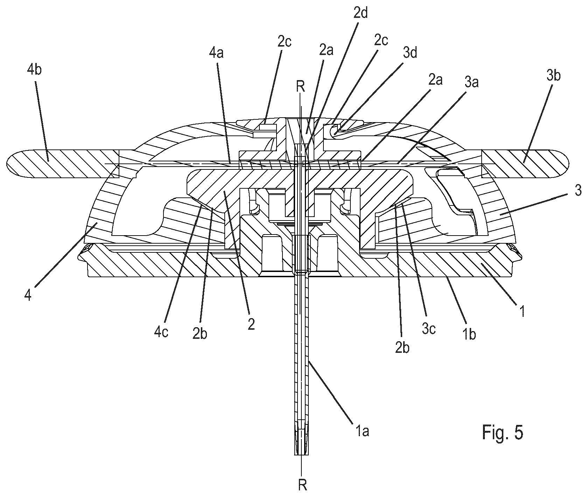

Das Infusionsset umfasst einen Basisteil (1). Der Basisteil (1) umfasst eine proximale und eine distale Seite. Der Basisteil (1) dient dem Anbringen des Infusionssets auf der Haut, wobei auf der distalen Seite des Basisteil ein Heftpflaster (1b) angeordnet ist. An dem Basisteil (1) ist zudem eine Kanüle (Figur 5; 1a) zur Zuführung des flüssigen Medikaments in den Körper des Patienten vorgesehen. Die Kanüle (Figur 5; 1a) kann als weiche oder steife Kanüle (Figur 5; 1a) ausgebildet sein. Das Infusionsset umfasst ferner einen Verbindungsteil (2). Der Verbindungsteil (2) ist an der proximalen Seite des Basisteils (1) untrennbar vorgesehen. Der Verbindungsteil (2) und der Basisteil (1) sind relativ zueinander um eine parallel zur Längsachse der Kanüle (Figur 5; 1a) verlaufenden Rotationsachse (R) drehbar angeordnet. Durch die relative Drehung um die Rotationsachse (R) kann der Tragekomfort verbessert werden, da die Bewegungsfreiheit des Patienten gewährleistet wird. Das Infusionsset weist einen ersten Konnektorteil (3) mit einer ersten Zuführungsleitung (3b), beispielsweise einem ersten Schlauch, auf. Die erste Zuführungsleitung (3b) dient zur Verbindung mit einer ersten Infusionsvorrichtung. Der erste Konnektorteil (3) weist eine erste Zuführungskanüle (3a) zur Verbindung mit dem Verbindungsteil (2) auf. Das Infusionsset umfasst ferner in dieser Ausführungsform der Erfindung einen zweiten Konnektorteil (4) auf, welcher einerseits eine zweite Zuführungsleitung (4b) zur Verbindung mit einer zweiten Infusionsvorrichtung und welcher andererseits eine zweite Zuführungskanüle (4a) zur Verbindung mit dem Verbindungsteil (2) umfasst. Der zweite Konnektorteil (4) kann als ergänzender Teil zu dem ersten Konnektorteil (3) ausgebildet sein. In einer anderen Ausführungsform der Erfindung kann der zweite Konnektorteil nur als ein ergänzender Teil zu dem ersten Konnektorteil (3) ausgestaltet sein und somit keine zweite Zuführungsleitung und keine zweite Zuführungskanüle umfassen. Diese beiden zweite Konnektorteile können nach Bedarf an den Verbindungsteil (2) des Infusionssets angekoppelt oder abgekoppelt werden. Der als ergänzender Teil ausgebildete zweite Konnektorteil ist derart ausgebildet, dass der erste (3) und der zweite Konnektorteil (4) im Wesentlichen den Basisteil (1) verdecken, wenn der erste (3) und der zweite Konnektorteil (4) in Eingriff sind. Wenn der erste (3) und der zweite Konnektorteil (4) in Eingriff sind, können zwei flüssige Produkte, insbesondere zwei flüssige Medikamente von den beiden Infusionsvorrichtungen in den Körper zugeführt werden kann. Die zwei flüssigen Produkte, insbesondere zwei Medikamente können gleichzeitig, hintereinander und/oder bei entsprechendem Bedarf in den Körper verabreicht werden, wobei eine Krankheit, beispielsweise Diabetes, Adipositas, Parkinson, Autoimmunerkrankungen, gezielter therapiert werden kann oder eine Therapie, beispielsweise eine Schmerztherapie oder eine Immuntherapie, gezielter behandelt werden kann.

Der erste Konnektorteil (3) und der zweite Konnektorteil (4) des erfindungsgemässen Infusionssets sind derart an den Verbindungsteil (2) ankoppelbar und abkoppelbar, dass jeweils eine lösbare Verbindung in eine

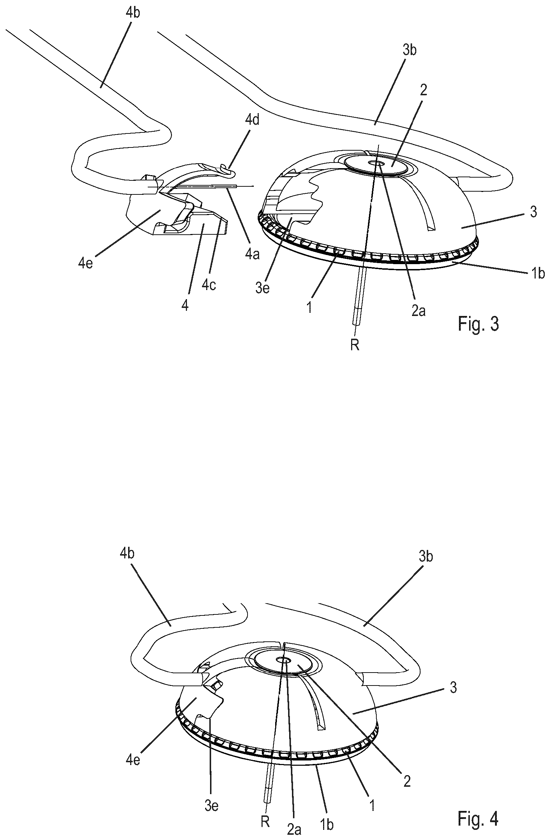

Richtung quer zur Längsachse der Kanüle (Figur 5; 1a) erfolgt. Durch diese Anordnung können die Schmerzen, welche durch die eingestochene Kanüle (Figur 5; 1a) des Basisteils (1) verursacht werden, verringert werden, da während der An- und Abkoppelung kein oder ein verringerter Druck auf die Einstichstelle wirkt. In der Figur 3 ist eine perspektivische Draufsicht auf das Infusionsset gemäss den Figuren 1 und 2 dargestellt, wobei ein erster Konnektorteil (3) mit dem Verbindungsteil (2) verbunden ist und der zweite Konnektorteil (4) von dem Verbindungsteil (2) entfernt ist. In der Figur 4 ist eine perspektivische Draufsicht und in der Figur 5 eine Längsschnittansicht auf das Infusionsset gemäss den Figuren 1 und 2 dargestellt, wobei der erste (3) und der zweite Konnektorteil (4) mit dem Verbindungsteil (2) verbunden sind.

Das Verbindungsteil (2) des Infusionssets weist ein Septum (2a) auf, welches einen parallel zur Längsachse der Kanüle (1a) verlaufenden Kanal (2d) aufweist, um eine Fluidverbindung zwischen dem Verbindungsteil (2) und der Kanüle (1a) in dem Basisteil (1) zu bilden. Das Septum (2a) erstreckt sich quer zur Längsachse der Kanüle (1a), so dass die erste Zuführungskanüle (3a) des ersten Konnektorteils (3) das Septum (2a) durchstossen kann, um eine Fluidverbindung zwischen dem ersten Konnektorteil (3) und dem Kanal (2d) des Verbindungsteils (2) zu bilden. Das Septum (2a) kann ein proximales Ende des Kanals (2d) verschliessen.

Der zweite Konnektorteil (4) umfasst die zweite Zuführungskanüle (4a) zur Verbindung mit dem Verbindungsteil (2) und umfasst die zweite Zuführungsleitung (4b) zur Verbindung mit einer zweiten Infusionsvorrichtung, wobei das Septum (2a) des Verbindungsteils (2) sich quer zur Längsachse der Kanüle (1a) erstreckt, so dass die zweite Zuführungskanüle (4a) des zweiten Konnektorteils (4) das Septum (2a) durchstösst, um eine Fluidverbindung zwischen dem zweiten Konnektorteil (4) und dem Kanal des Verbindungsteils zu bilden.

Der Basisteil (1) und der Verbindungsteil (2) können in einer Ausführungsform der Erfindung relativ zueinander um die Rotationsachse (R) um 360° oder in einer anderen Ausführungsform der Erfindung um die Rotationsachse (R) um weniger als 360° drehbar angeordnet sein.

Der erste Konnektorteil (3) und der zweite Konnektorteil (4) können an den Verbindungsteil (2) derart ankoppelbar und abkoppelbar sein, dass jeweils eine lösbare, insbesondere eine lösbare axialfeste und eine lösbare, insbesondere eine lösbare drehfeste Verbindung zwischen dem ersten Konnektorteil (3) und dem zweiten Konnektorteil (4) und an den Verbindungsteil (2) erfolgen kann.

Das Verbindungsteil (2) weist ein Rastelement (2c) in Form einer Ringnut auf, welches mit einem an dem ersten Konnektorteil vorgesehenen ersten korrespondierenden Rastgegenelement (3d) in Form eines ersten Rastrings eine lösbare, insbesondere lösbare axialfeste Verbindung bilden kann und welches mit einem an dem zweiten Konnektorteil (4) vorgesehenen zweiten korrespondierenden Rastgegenelement (4d) in Form eines zweiten Rastrings eine lösbare, insbesondere lösbare axialfeste Verbindung bilden kann. Die Rastverbindung dient insbesondere dazu, dass der erste (3) und der zweite Konnektorteil (4) lösbar, insbesondere lösbar axialfest an den Verbindungsteil (2) ankoppelbar und abkoppelbar sind. Es können auch andere lösbare, insbesondere lösbar axialfeste Verbindungen zwischen dem Verbindungsteil (2) und dem ersten (3) und dem zweiten Konnektorteil (4) vorgesehen sein.

Das Verbindungsteil (2) weist ein Führungselement (2b) auf, welches mit einem an dem ersten Konnektorteil (3) vorgesehenen ersten korrespondierenden Führungsgegenelement (3c) in Eingriff gelangen kann und welches mit einem an dem zweiten Konnektorteil (4) vorgesehenen zweiten korrespondierenden Führungsgegenelement (4c) in Eingriff gelangen kann. Die Führungseingriffe können als lösbare, insbesondere als lösbare drehfeste Verbindungen ausgebildet sein. Diese Führungseingriffe dienen insbesondere dazu, dass der erste und der zweite Konnektorteil lösbar, insbesondere lösbar drehfest an den Verbindungsteil ankoppelbar und abkoppelbar sind. Die Ankoppelung und Abkoppelung zwischen dem ersten (3) bzw. dem zweiten Konnektorteil (4) und dem Verbindungsteil (2) erfolgt derart, dass jeweils eine lösbare, insbesondere lösbare drehfeste Verbindung um die Rotationsachse (R) erfolgen kann.

Das Führungselement (2b) des Verbindungsteils (2) ist zumindest teilweise polygonförmig ausgebildet. Ferner sind das erste Führungsgegenelement (3c) des ersten Konnektorteils (3) und das zweite Führungsgegenelement (4c) des zweiten Konnektorteils (4) als korrespondierender Teil zu diesem teilweisen polygonförmigen Verbindungsteil ausgebildet. Das Verbindungsteil (2) hat einen polygonförmigen Querschnitt. Das Polygon kann n-Ecken aufweisen. In diesem Ausführungsbeispiel der Erfindung weist das Polygon acht Ecken auf, wobei auch eine andere Anzahl vorgesehen werden kann. Das Polygon ist als regelmässiges Polygon ausgebildet. Alternativ kann das Polygon unregelmässig ausgebildet sein. Die Seiten des Polygons können parallel und/oder schräg zur Längsachse der Kanüle (1a) ausgebildet sein. Die Seiten des Polygons dienen als Führung.

### Bezugszeichen:

- 1: Basisteil
- 1a: Kanüle
- 1b: Heftpflaster
- 2: Verbindungsteil
- 2a: Septum
- 2b: Führungselement
- 2c: Rastelement
- 2d: Kanal
- 3: erste Konnektorteil
- 3a: erste Zuführungskanüle
- 3b: erste Zuführungsleitung
- 3c: erste Führungsgegenelement
- 3d: erste Rastgegenelement
- 3e: Ausnehmung
- 4: zweite Konnektorteil
- 4a: zweite Zuführungskanüle
- 4b: zweite Zuführungsleitung
- 4c: zweite Führungsgegenelement
- 4d: zweite Rastgegenelement
- 4e: Verschlusselement
- R: Rotationsachse

## Patentansprüche

1. Infusionsset mit
- einem Basisteil (1) mit einer proximalen und einer distalen Seite, wobei an der distalen Seite des Basisteils (1) eine Kanüle (1a) zur Zuführung eines flüssigen Medikaments in den Körper des Patienten vorgesehen ist,
- ein Verbindungsteil (2), wobei der Verbindungsteil (2) an der proximalen Seite des Basisteils (1) untrennbar vorgesehen ist und wobei der Verbindungsteil (2) und das Basisteil (1) relativ zueinander um eine parallel zur Längsachse der Kanüle (1a) verlaufenden Rotationsachse (R) drehbar angeordnet sind,
- einem ersten Konnektorteil (3) mit einer ersten Zuführungskanüle (3a) zur Verbindung mit dem Verbindungsteil (2) und mit einer ersten Zuführungsleitung (3b) zur Verbindung mit einer ersten Infusionsvorrichtung,
- einem zweiten Konnektorteil (4),
- wobei der erste Konnektorteil (3) und der zweite Konnektorteil (4) an den Verbindungsteil (2) derart ankoppelbar und abkoppelbar sind, dass jeweils eine lösbare Verbindung in eine Richtung quer zur Längsachse der Kanüle (1a) erfolgt,
- **dadurch gekennzeichnet, dass** der Verbindungsteil (2) ein Septum (2a) umfasst, welches einen parallel zur Längsachse der Kanüle (1a) verlaufenden Kanal (2d) aufweist, um eine Fluidverbindung zwischen dem Verbindungsteil (2) und der Kanüle (1a) in dem Basisteil (1) zu bilden, und welches sich quer zur Längsachse der Kanüle (1a) erstreckt, so dass die erste Zuführungskanüle (3a) des ersten Konnektorteils (3) das Septum (2a) durchstossen kann, um eine Fluidverbindung zwischen dem ersten Konnektorteil (3) und dem Kanal (2d) des Verbindungsteils (2) zu bilden.

2. Infusionsset nach Anspruch 1, **dadurch gekennzeichnet, dass** der erste Konnektorteil (3) und der zweite Konnektorteil (4) an den Verbindungsteil (2) derart ankoppelbar und abkoppelbar sind, dass eine lösbare axialfeste und/oder eine lösbare drehfeste Verbindung zwischen dem ersten Konnektorteil (3) und dem Verbindungsteil (2) und zwischen dem zweiten Konnektorteil (4) und dem Verbindungsteil (2) erfolgen kann.

3. Infusionsset nach Anspruch 1 oder 2, **dadurch gekennzeichnet, dass** an dem zweiten Konnektorteil (4) eine zweite Zuführungskanüle (4a) zur Verbindung mit dem Verbindungsteil (2) und eine zweite Zuführungsleitung (4b) zur Verbindung mit einer zweiten Infusionsvorrichtung vorgesehen ist und wobei das Septum (2a) des Verbindungsteils (2) sich quer zur Längsachse der Kanüle (1a) erstreckt, so dass die zweite Zuführungskanüle (4a) des zweiten Konnektorteils (4) das Septum (2a) durchstossen kann, um eine Fluidverbindung zwischen dem zweiten Konnektorteil (4) und dem Kanal (2d) des Verbindungsteils (2) zu bilden.

4. Infusionsset nach einem der vorhergehenden Ansprüche, **dadurch gekennzeichnet, dass** der Basisteil (1) und der Verbindungsteil (2) relativ zueinander um die parallel zur Längsachse der Kanüle (1a) verlaufenden Rotationsachse (R) um 360° oder um weniger als 360° drehbar angeordnet sind.

5. Infusionsset nach einem der vorhergehenden Ansprüche, **dadurch gekennzeichnet, dass** der Verbindungsteil (2) ein Rastelement (2c) aufweist, welches mit einem an dem ersten Konnektorteil (3) vorgesehenen ersten korrespondierenden Rastgegenelement (3d) eine lösbare Verbindung, insbesondere eine lösbare axialfeste Verbindung bilden kann und welches mit einem an dem zweiten Konnektorteil (4) vorgesehenen zweiten korrespondierenden Rastgegenelement (4d) eine lösbare Verbindung, insbesondere eine lösbare axialfeste Verbindung bilden kann.

6. Infusionsset nach einem der vorhergehenden Ansprüche, **dadurch gekennzeichnet, dass** der Verbindungsteil (2) ein Führungselement (2b) aufweist, welches mit einem an dem ersten Konnektorteil (3) vorgesehenen ersten korrespondierenden Führungsgegenelement (3c) in Eingriff gelangen kann, um eine lösbare Verbindung, insbesondere eine lösbare drehfeste Verbindung um die Rotationsachse (R) zu bilden und welches mit einem an dem zweiten Konnektorteil (4) vorgesehenen zweiten korrespondierenden Führungsgegenelement (4c) in Eingriff gelangen kann, um eine lösbare Verbindung insbesondere eine lösbare drehfeste Verbindung um die Rotationsachse (R) zu bilden.

7. Infusionsset nach Anspruch 6, **dadurch gekennzeichnet, dass** der erste (3) und der zweite Konnektorteil (4) derart in Eingriff mit dem Verbindungsteil (2) sein können, dass sie formschlüssig lösbar ineinandergreifen können.

8. Infusionsset nach Anspruch 6 oder 7, dass das Führungselement (2d) des Verbindungsteils (2) zumindest teilweise polygonförmig ausgebildet ist und das erste Führungsgegenelement (3c) des ersten Konnektorteils (3) und das zweite Führungsgegenelement (4c) des zweiten Konnektorteils (4) als korrespondierende Teile zu diesem teilweisen polygonförmigen Verbindungsteil (2) ausgebildet sind.

9. Infusionsset nach Anspruch 8, **dadurch gekennzeichnet, dass** der Basisteil (1) kreisförmig ausgebildet ist, und wenn der erste (3) und der zweite Konnektorteil (4) formschlüssig lösbar ineinandergreifen, den Basisteil (1) im Wesentlichen verdeckt.

10. Infusionsset nach einem der vorhergehenden Ansprüche, **dadurch gekennzeichnet, dass** das Septum (2a) ein proximales Ende des Kanals (2d) verschliesst.
